# EUROPEAN PATENT SPECIFICATION

(11) **EP 4 029 509 B1**
(45) Date of publication and mention of the grant of the patent: **04.11.2026**
(21) Application number: 20862447.8
(22) Date of filing: 10.09.2020
(51) Int. Cl.: A61K 36/06, A61K 31/716, A61K 39/395, A23L 33/10, A61P 35/00, A61P 43/00, A61K 45/06, A61K 39/00, C07K 16/28

(54) **BETA-GLUCANS FROM AUREOBASIDIUM PULLULANS FOR ENHANCING ANTI-CANCER EFFECT OF ANTIBODY DRUGS**
BETA-GLUCANE VON AUREOBASIDIUM PULLULANS ZUR ERHÖHUNG DER ANTIKREBS WIRKUNG EINES ANTIKÖRPERMEDIKAMENTS
BETA-GLUCANS DE AUREOBASIDIUM PULLULANS POUR AUGMENTER L'EFFET ANTICANCÉREUX D'UN MÉDICAMENT DE TYPE ANTICORPS

(30) Priority: 11.09.2019 JP 2019165722
(43) Date of publication of application: 20.07.2022
(73) Proprietor: Aureo Co., Ltd., Kimitsu-shi, Chiba, 292-1149 (JP); National Center for Child Health and Development, Tokyo 157-8535 (JP)
(72) Inventor: RII Ko, Tokyo 157-8535 (JP); FUJINO Masayuki, Tokyo 157-8535 (JP); MORIYA Yukiko, Kimitsu-shi, Chiba 292-1149 (JP)
(74) Representative: Dehns
(86) International application number: PCT/JP2020/034361
(87) International publication number: WO 2021/049587

(56) References cited:
- WO-A1-2006/027914
- WO-A1-2016/007876
- WO-A1-2017/120604
- WO-A1-2021/256470
- WO-A2-2016/073763
- JP-A- 2002 204 687
- JP-A- 2017 524 737
- CLARK CURTIS A., GUPTA HARSHITA B., SAREDDY GANGADHARA, PANDESWARA SRILAKSHMI, LAO SHUNHUA, YUAN BIN, DRERUP JUSTIN M., PADRON ALV: "Tumor-intrinsic PD-L1 signals regulate cell growth, pathogenesis, and autophagy in ovarian cancer and melanoma", CANCER RES., vol. 76, no. 23, 1 December 2016 (2016-12-01), pages 6965, XP055806131
- KIMURA, YOSHIYUKI ET AL.: "P-1197: Antitumor and antimetastatic effects of beta-glucan in colon 26-implanted mice", CANCER SCIENCE, vol. 95, no. Suppl., 2004, pages 456, XP009532971
- YAMASAKI, KAORI ET AL.: "3A07p24 Antitumor effect by oral administration of extracellular beta-glucan of black yeast", ANNUAL MEETING OF THE JAPAN SOCIETY OF BIOSCIENCE, BIOTECHNOLOGY, AND AGROCHEMISTRY, 2004 (JSBBA 2004), vol. 2004, 5 March 2004 (2004-03-05), pages 165, XP009532931

## Description

### TECHNICAL FIELD

The present invention relates to a composition for enhancing the effect of an antibody drug which has an effect of suppressing growth of cancer via immune checkpoint inhibition.

### BACKGROUND ART

Molecular targeted drugs that target immune checkpoints are attracting attention as novel cancer treatment methods. Immune checkpoints are a braking mechanism to prevent runaway so that activated immune cells do not attack normal tissues or cells; cancer cells, on the contrary, utilize this mechanism to avoid the attack of immune cells. This is one of the functions cancer cells use for their own survival, and there are aspects that have yet to be possible to clarify. As research in this field has progressed, it has been clarified in recent years that effective anti-cancer effects are obtained even in clinical practice by antibody drugs, etc., that inhibit the function of PD-1 and CTLA4, which are immune checkpoint molecules (see Non-Patent Documents 1-3).

On the other hand, the present applicant has disclosed utilizing *Aureobasidium pullulans,* also known as black yeast, which produces β-glucans abundantly, as a moisturizer for skin (Patent Document 1), an agent to improve constipation (Patent Document 2), an immunopotentiator (Patent Document 3), an immunoadjuvant (Patent Document 4), an agent to promote body healing used to reduce the side effects of anticancer drugs (Patent Document 5), an agent to promote body healing used to promote healing of burns (Patent Document 6), a composition for the prevention/treatment of mastitis in cows (Patent Document 7), a composition to promote cytokine production in macrophages (Patent Document 8), an agent for treatment of influenza virus infection (Patent Document 9), a TRAIL expression enhancer (Patent Document 10), a composition for preventing/improving anemia (Patent Document 11), an agent for suppressing fat accumulation (Patent Document 12), etc.

### [Related Art Documents]

### [Non-Patent Documents]

[Non-Patent Document 1] Ishida, Y., Agata, Y., Shibahara, K. and Honjo, T. "Induced expression of PD-1, a novel member of the immunoglobulin gene superfamily, upon programmed cell death." EMBO J. 11 3887-3895 (1992)
[Non-Patent Document 2] Freeman, G. J., Long, A. J., Iwai, Y., Borque, K., Chernova, T., Nishimura, H., Fitsz, L. J., Malenkovich, N., Okazaki, T., Byrne, M. C., Horton, H. F., Fouser, L., Carter, L., Ling, V., Bowman, M. R., Carreno, B. M., Collins, M., Wood, C. R. and Honjo, T. "Engagement of the PD-1 immunoinhibitory receptor by a novel B7 family member leads to negative regulation of lymphocyte activation. J. Exp. Med. 192 1027-1034 (2000)
[Non-Patent Document 3] Spencer C. Wei, Colm R. Duffy, and James P. Allison "Fundamental Mechanisms of Immune Checkpoint Blockade Therapy" CANCER RESEARCH, doi: 10.1158/2159-8290.CD-18-0367, August 16, 2018

### [Patent Documents]

[Patent Document 1] Japanese Patent No. 4000078
[Patent Document 2] Japanese Patent No. 4054697
[Patent Document 3] Japanese Patent No. 4369258
[Patent Document 4] Japanese Patent No. 5242855
[Patent Document 5] Japanese Patent No. 5331482
[Patent Document 6] Japanese Patent No. 5715659
[Patent Document 7] Japanese Patent No. 5554221
[Patent Document 8] Japanese Patent No. 5559173
[Patent Document 9] Japanese Patent No. 5560472
[Patent Document 10] Japanese Patent No. 5937029
[Patent Document 11] Japanese Patent No. 6293187
[Patent Document 12] Japanese Patent No. 6380968 WO 2016/007876 A1, WO 2017/120604 A1 and WO 2016/073763 A2 disclose combinations of β-glucan derived from yeast with immune checkpoint inhibitors for treating cancers.
JP 2002 204687 A discloses discloses β-glucan derived from Aureobasidium pullulans suppressing cancer cell growth, e.g. leukemia and skin cancer.

### DISCLOSURE OF THE INVENTION

### [Problems the Invention is Intended to Solve]

An object of the present invention is to utilize *Aureobasidium pullulans* to provide a composition capable of enhancing the effect of an antibody drug such as an immune checkpoint inhibitor, etc.

### [Means for Solving the Problems]

Upon having conducted thoroughgoing studies into achieving the above goal, the present inventors perfected the invention by discovering that the β-glucans contained in cultures of *Aureobasidium pullulans* act to enhance the effect of antibody drugs such as immune checkpoint inhibitors, etc., when used in combination therewith. Specifically, the present invention is as defined in the claims.

### [Effect of the Invention]

In the present invention, a β-glucan derived from *Aureobasidium pullulans* is used as an active ingredient; therefore, the effect of the antibody drug which has an effect of suppressing growth of cancer via immune checkpoint inhibition can be enhanced by administering together with an antibody drug, allowing suitable use as a potentiator of an immune checkpoint inhibitor. in the treatment of cancer.

### BRIEF DESCRIPTION OF THE DRAWINGS

FIG. 1 is a chart showing an outline of the administration schedule of each test substance in Test Example 1.
FIG. 2 is a chart showing the results of studying tumor volume after a predetermined number of days from tumor cell transplantation in Test Example 1.
FIG. 3 is a chart showing the results of FACS analysis of spleen lymphocytes after multiple staining by anti-mouse CD4 antibody or instead anti-mouse CD8 antibody and anti-mouse Ki-67 (lymphocyte activation marker) antibody in Test Example 2.
FIG. 4 is a chart showing the results of FACS analysis of tumor infiltrating lymphocytes (TIL) after multiple staining by anti-mouse CD4 antibody or instead anti-mouse CD8 antibody and anti-mouse Ki-67 (lymphocyte activation marker) antibody in Test Example 2.
FIG. 5 is a chart showing the results of FACS analysis of spleen lymphocytes after multiple staining by anti-mouse CD4 antibody or instead anti-mouse CD8 antibody and anti-mouse INF-γ antibody in Test Example 2.
FIG. 6 is a chart showing the results of FACS analysis of tumor infiltrating lymphocytes after multiple staining by anti-mouse CD4 antibody or instead anti-mouse CD8 antibody and anti-mouse INF-γ antibody in Test Example 2.
FIG. 7 is a showing an outline of the administration schedule of each test substance in Test Example 3.
FIG. 8 is a chart showing the results of studying tumor volume after a predetermined number of days from tumor cell transplantation in Test Example 3.

### MODE FOR CARRYING OUT THE INVENTION

In the present invention, a β-glucan derived from *Aureobasidium pullulans* is used as an active ingredient of a composition for enhancing the effect of an antibody drug.

β-Glucans are polysaccharides in which β-glucose is polymerized by glycoside bonds. Since β-glucans are contained, for example, in barley, oats, and other such cereals; brewer's yeast, baker's yeast, and other such yeasts; black yeast and other such imperfect fungi; shiitake (Lentinula edodes), maitake (Grifola frondosa), turkey tail mushroom (Coriolus versicolor), split gill mushroom (Schizophyllum commune), cauliflower mushroom (Sparassis crispa), and other such basidiomycetes; and kelp, wakame, and other such seaweeds, β-glucans can be obtained by extraction from these natural materials using a suitable solvent, etc. Usual methods are acceptable as the extraction method and are not particularly restricted. For example, water, hydrous alcohol, or another such extraction solvent is added to a raw material that has been dried and crushed as needed, and extraction is performed under heating and/or pressurization. Heating conditions are typically 105-135°C, and pressurization conditions are typically 1.8-2.1 atm. Extraction may also be carried out while performing acid treatment or alkali treatment or enzyme treatment by an enzyme that lowers the molecular weight of the polysaccharide, to improve the extraction efficiency. After extraction, the solvent may be distilled off to effect concentration, and the product may be dried and converted to a pulverulent form by a drying means such as spray drying. Various β-glucan materials have entered the marketplace in recent years, and such commercial products therefore may be utilized.

The β-glucan used in the present invention is preferably polymerized by β-1,3 glycoside bonds, β-1,4 glycoside bonds, β-1,6 glycoside bonds, etc., more preferably includes homopolymerization of β-1,3 glycoside bonds as the main chain or includes heteropolymerization of β-1,3 glycoside bonds and β-1,6 glycoside bonds as the main chain, and even more preferably includes homopolymerization of β-1,3 glycoside bonds as the main chain and includes β-1,6 glycoside bonds as side chains. β-Glucans may have functional groups such as sulfuric acid groups and phosphoric acid groups. An average molecular weight range of from 5,000 to 1,000,000 is preferred as the molecular weight, and an average molecular weight range of from 200,000 to 500,000 is more preferred. The molecular weight of β-glucans can be measured by gel filtration method, etc.

The β-glucan used in the present invention, as will be shown in the examples below, is a β-glucan-containing culture composition derived from a culture of *Aureobasidium pullulans.*

β-Glucans derived from microorganisms belonging to Aureobasidium sp. will be described in greater detail below. Culture compositions containing such β-glucans include not only cultures obtained by culturing microorganisms belonging to *Aureobasidium pullulans* (sometimes referred to hereinafter as Aureobasidium microorganisms), cultures obtained by separating and removing cells by centrifugation, etc., concentrated solutions of said cultures, diluted solutions of said cultures, and solid products obtained by removing the water content from said cultures, but also cultures containing greater amounts of specific components such as β-glucans by desalting or otherwise processing said cultures.

The Aureobasidium microorganisms used in the present invention may be any microorganism belonging to *Aureobasidium pullulans* and having the ability to produce β-glucans; e.g., *Aureobasidium pullulans* M-1 (deposit number: FERM BP-08615, deposit date: February 10, 2004, transferred from FERM P-19213 deposited on February 14, 2003) (National Institute of Technology and Evaluation, International Patent Organism Depository, Room 120, 2-5-8 Kazusakamatari, Kisarazu-shi, Chiba-ken, Japan, postal code 292-0818) and *Aureobasidium pullulans* M-2 (deposit number FERM BP-10014, deposit date: April 22, 2004) (National Institute of Technology and Evaluation, International Patent Organism Depository, Room 120, 2-5-8 Kazusakamatari, Kisarazu-shi, Chiba-ken, Japan, postal code 292-0818) is suited for use. Furthermore, structural analysis performed using NMR measurement (13C NMR: model UNITY INOVA 500 manufactured by Varian, 1H NMR: model UNITY INOVA 600 manufactured by Varian) makes clear that the β-glucan produced by these strains is β-1,3-1,6-glucan having a structure in which glucose branches by β-1,6 bonds from a main chain of β-1,3-bonded glucose.

The Aureobasidium microorganisms can be cultured in accordance with a known method (see JP (Kokai) Sho 57-149301, etc.). Specifically, medium (pH 5.2-6.0) to which a carbon source (sucrose) 0.5-5.0 mass%, nitrogen source (e.g., rice bran) 0.1-5.0 mass%, and other trace substances (e.g., vitamins, inorganic matter) have been added may be inoculated with fungus and aeration-cultured, preferably aeration-stir-cultured, for 2-14 days at 20-30°C. The viscosity of the culture rises as β-glucan is produced, resulting in a thick, viscous gel. The resulting culture usually includes 0.6-10 mass% of solids, and the solids include 5-80 mass% of β-glucan.

The culture that includes a β-glucan obtained by culturing as described above is preferably sterilized by heating or by pressurization and heating for use. The culture may also be sterilized for use after separating and removing the cells by centrifugation, etc. A culture that has been concentrated or dried as needed can also be used. A culture obtained using an extracted component rich in β-glucans or a desalted, refined product thereof can also be used. Furthermore, cultures of microorganisms belonging to *Aureobasidium pullulans* are used as food additives such as thickening stabilizers and are highly safe.

Due to enhancing the effect of antibody drugs, the composition according to the present invention is administered together with an antibody drug on use. In other words, when the effect of an antibody drug is expressed for the purpose of an anticancer effect, the composition can be used to heighten that effect.

By "administered together with an antibody drug" is meant that both the above β-glucan or β-glucan-containing culture composition and the antibody drug are brought into contact with tissue, cells, or another biological structure that is the target of application in a timely manner within a range that shows their efficacy, it not being necessary that the two are administered in the form of a single formulation. That is, the effect of the present invention can be enjoyed even if the two components are administered continuously, each as separate formulations, or administered at predetermined intervals, within a range that shows their efficacy.

The antibody drug to which the composition according to the present invention is applied is an antibody that acts to suppress the growth of a cancer via immune checkpoint inhibition. There are no particular limitations as to the types of cancers to which antibody drugs can be applied. Examples include lung cancer, lymphoma, melanoma, leukemia, kidney cell carcinoma, renal pelvis/ureteral cancer, pharyngeal cancer, osteosarcoma, stomach cancer, malignant mesothelioma, ovarian cancer, cervical cancer, pancreatic cancer, MSI-High colon/rectal cancer, esophageal cancer, hepatocellular carcinoma, biliary tract cancer, etc. Anti-PD-L1 antibodies, anti-PD-1 antibodies, anti-LAG3 antibodies, anti-CTLA4 antibodies, anti-TIM3 antibodies, anti-TIGIT antibodies, anti-VISTA antibodies, etc., for example, are known as antibodies, but are not limited to these. The antibody may be a polyclonal antibody, a monoclonal antibody, or in the form of antiserum including an antibody. The antibody may also be an antibody to a human protein or an antibody to a protein of an animal such as a mouse, rat, rabbit, goat, cow, monkey, etc. In addition, the antibody may be a human type antibody or an animal type antibody such as a mouse. Alternatively, the antibody may be prepared by producing an antiserum in an animal such as a mouse, rat, rabbit, goat, cow, monkey, etc.

There is no particular limitation as to the administration route for the composition according to the present invention; known formulation forms can be selected as is appropriate and applied by oral administration, intraperitoneal administration, intramuscular administration, nasal administration, pulmonary administration, vaginal administration, intravenous administration, rectal administration, etc.

The dosage of the composition according to the present invention can be decided as is appropriate depending on the type of antibody drug, health status of the person or animal being administered, symptoms, age, administration method, frequency, time, etc. To give a general example of dosage, in the case of oral intake, the β-glucan or β-glucan-containing culture composition is administered in an amount of 0.025-4000 mg/kg (body weight) in terms of solids. In the case of intraperitoneal administration, the β-glucan or β-glucan-containing culture composition is administered in an amount of 0.05-5 mg/kg (body weight) in terms of solids.

The composition according to the present invention can typically be used in various formulation forms; e.g., as a drug product, topical drug product, functional food, nutritional supplement, supplement, health food, veterinary drug product, veterinary topical drug product, veterinary functional food, veterinary nutritional supplement, veterinary supplement, veterinary health food, etc. Alternatively, these products can be used in combination or in combination with various types of foods and beverages.

### [Examples]

The present invention is described concretely below through examples, but these examples do not limit the scope of the present invention.

### [Production example 1]

A β-glucan was produced in culture broth by inoculating liquid medium with *Aureobasidium pullulans* M-2 (National Institute of Technology and Evaluation, International Patent Organism Depository deposit number: FERM BP-10014) and shake culturing for four days at 24.5°C. The β-glucan concentration was estimated to be approximately 6 mg/mL by the phenol-sulfuric acid method and enzyme method.

### <Test example 1>

Cancer-bearing mice were created by transplantation of a tumor cell line in accordance with the conventional method, and whether administration of the test substance affected growth of the tumors was studied.

Specifically, cancer-bearing mice were created by inoculating the flanks of C57BL/6 mice (Japan SLC) with 3×10⁵ mouse melanoma cell line B16F10 (Riken BioBank) cells and allowing the tumors to grow for 16 days.

The test animals were divided into four groups: an untreated group, an anti-PD-L1 antibody alone group, a β-glucan alone group, and an anti-PD-L1 antibody and β-glucan combined group. The N number was 26 animals in the untreated group, 18 animals in the anti-PD-L1 antibody alone group, 6 animals in the β-glucan alone group, and 9 animals in the anti-PD-L1 antibody and β-glucan combined group. Below, for the sake of convenience, the untreated group is sometimes referred to as the "control group," the anti-PD-L1 antibody alone group as "test group 1," the β-glucan alone group as "test group 2," and the anti-PD-L1 antibody and β-glucan combined group as "test group 3."

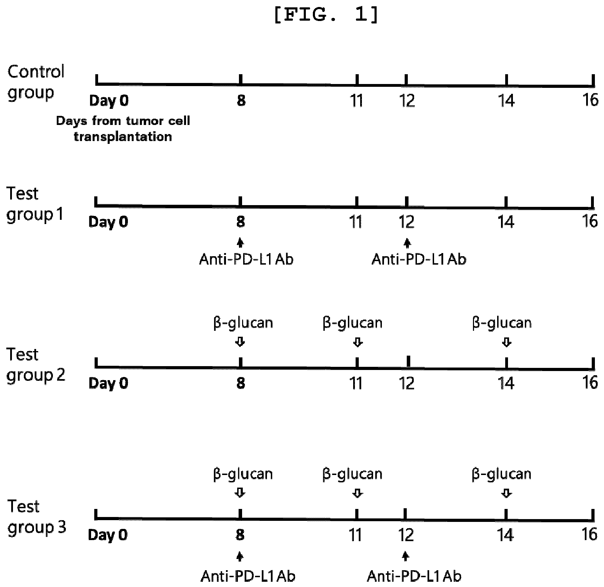

As the form of the test substance administered, in the anti-PD-L1 antibody alone group ("test group 1"), a dosage of 200 µg/animal of anti-mouse PD-L1 antibody (clone: MIH5) was administered intraperitoneally on days 8 and 12 after tumor cell transplantation. In the β-glucan alone group ("test group 2"), a dosage of 30 mg/kg in terms of β-glucan amount of the β-glucan-containing culture composition prepared in Production example 1 above was administered intraperitoneally on days 8, 11, and 14 after tumor cell transplantation. In the anti-PD-L1 antibody and β-glucan combined group ("test group 3"), anti-mouse PD-L1 antibody and the β-glucan-containing culture composition prepared in Production example 1 above were administered to the mice intraperitoneally in the same dosages as in the respective alone groups at the same times as in the respective alone groups. FIG. 1 shows an overview of the administration schedule of each test substance.

The major and minor axes of the tumors were measured on days 6, 8, 12, 14, and 16 after inoculation of the melanoma cell line, and the tumor volume (in cubic millimeters) determined via the following formula was recorded. [Numerical formula 1] $Tumor volume \left({mm}^{3}\right) = \left.major axis \left(mm\right)\times {\left(minor axis \left(mm\right)\right)}^{2}\right) / 2$

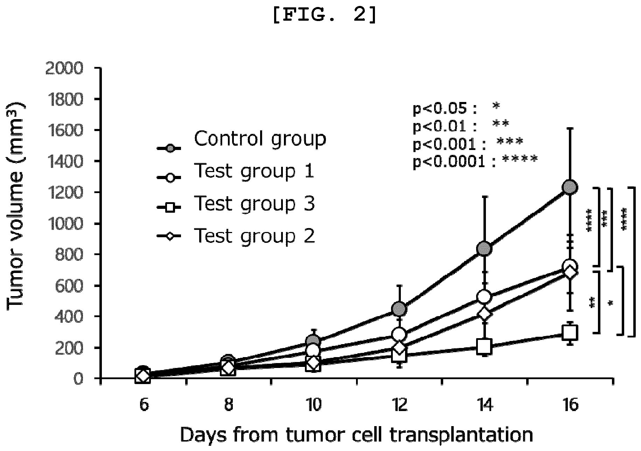

The results on tumor volume after the elapse of the predetermined number of days from tumor cell transplantation are shown in Table 1 and FIG. 2. The results obtained by studying the existence of significance in each group by statistical analysis using the Brown-Forsythe test when comparing tumor volumes on day 16 after tumor cell transplantation are shown in Table 2.

**[Table 1]**

| | | Tumor volume (mm³) after elapse of number of days from tumor cell transplantation | | | | | |
|---|---|---|---|---|---|---|---|
| | | Day 6 | Day 8 | Day 10 | Day 12 | Day 14 | Day 16 |
| Control group | Mean | 31.28 | 103.36 | 235.27 | 442.66 | 830.32 | 1228.20 |
| | S.D | 15.492 | 48.997 | 75.191 | 154.490 | 333.702 | 372.835 |
| Test group 1 | Mean | 24.78 | 81.86 | 174.60 | 278.79 | 521.86 | 717.79 |
| | S.D | 9.931 | 19.353 | 72.357 | 98.404 | 164.762 | 165.027 |
| Test group 2 | Mean | 16.25 | 69.13 | 102.29 | 197.50 | 415.86 | 681.78 |
| | S.D | 5.671 | 26.833 | 36.520 | 97.133 | 200.289 | 242.078 |
| Test group 3 | Mean | 13.75 | 65.73 | 93.56 | 150.55 | 202.14 | 290.79 |
| | S.D | 4.289 | 27.124 | 44.892 | 75.978 | 54.145 | 73.816 |

**[Table 2]**

| Existence of significance in each group when comparing tumor volumes on day 16 after tumor cell transplantation | | | |
|---|---|---|---|
| | Significance | Indication | Adjusted p value |
| Control group vs test group 1 | Yes | **** | <0.0001 |
| Control group vs test group 2 | Yes | *** | 0.0004 |
| Control group vs test group 3 | Yes | **** | <0.0001 |
| Test group 1 vs test group 2 | No | - | 0.9905 |
| Test group 1 vs test group 3 | Yes | ** | 0.003 |
| Test group 2 vs test group 3 | Yes | * | 0.0402 |

In the results, the tumor volume was markedly reduced in the anti-PD-L1 antibody and β-glucan combined group ("test group 3") in comparison to the untreated group ("control group"). The reduction effect was also statistically significant in comparison to the anti-PD-L1 antibody alone group ("test group 1") and the β-glucan alone group ("test group 2") (see comparison on day 16).

### <Test example 2>

The cancer-bearing mice were necropsied on day 16 after tumor cell transplantation, and spleen lymphocytes and tumor infiltrating lymphocytes (TIL) were separated and purified from cancer-bearing mice of each group. Lymphocytes were prepared using a lymphocyte separation kit (product name "Lympholyte-M", Cedarlane Laboratories) after hemolyzing the collected tissue by sterilized distilled water.

The lymphocytes were stained using anti-mouse CD4 antibody (BioLegend Japan K.K.), anti-mouse CD8 antibody (BioLegend Japan K.K.), anti-mouse Ki-67 (lymphocyte activation marker) antibody (Thermo Fisher Scientific Co., Ltd.), and anti-mouse INF-γ antibody (Becton Dickinson Japan), and FACS analysis was performed. Intracellular antigens were stained after staining the cell surface antigens. Staining of intracellular antigens used Fixation Buffer and Permeabilization Wash Buffer (BioLegend Japan K.K.).

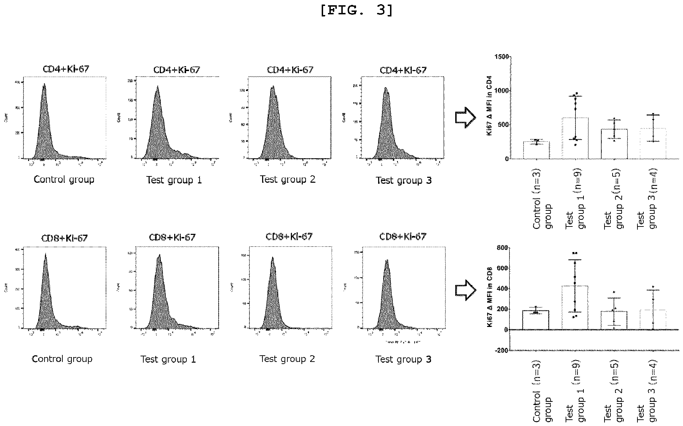

FIG. 3 shows the results of FACS analysis of spleen lymphocytes after multiple staining by anti-mouse CD4 antibody or instead anti-mouse CD8 antibody and anti-mouse Ki-67 (lymphocyte activation marker) antibody.

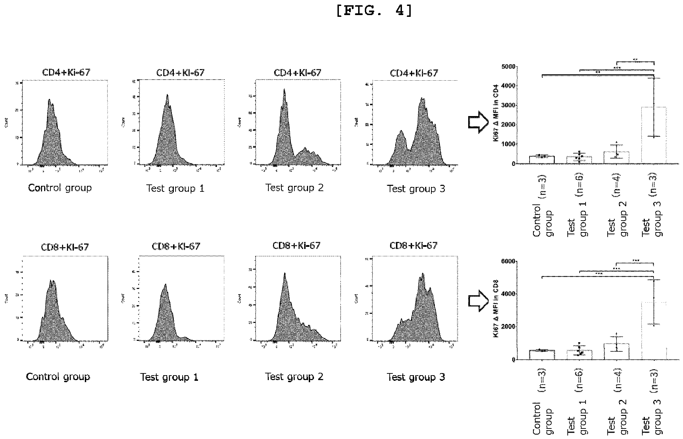

FIG. 4 shows the results of FACS analysis of tumor infiltrating lymphocytes (TIL) after multiple staining by anti-mouse CD4 antibody or instead anti-mouse CD8 antibody and anti-mouse Ki-67 (lymphocyte activation marker) antibody.

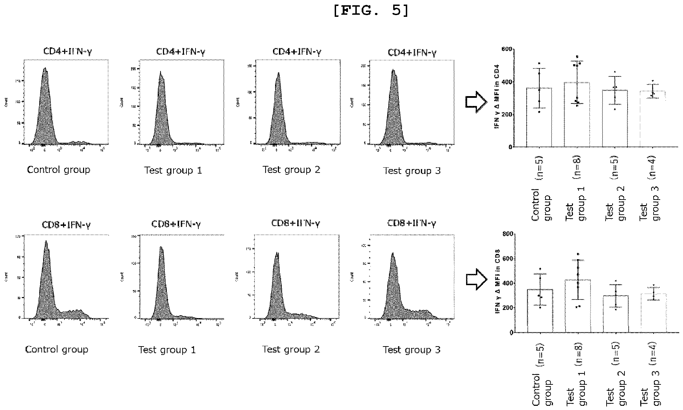

FIG. 5 shows the results of FACS analysis of spleen lymphocytes after multiple staining by anti-mouse CD4 antibody or instead anti-mouse CD8 antibody and anti-mouse INF-γ antibody.

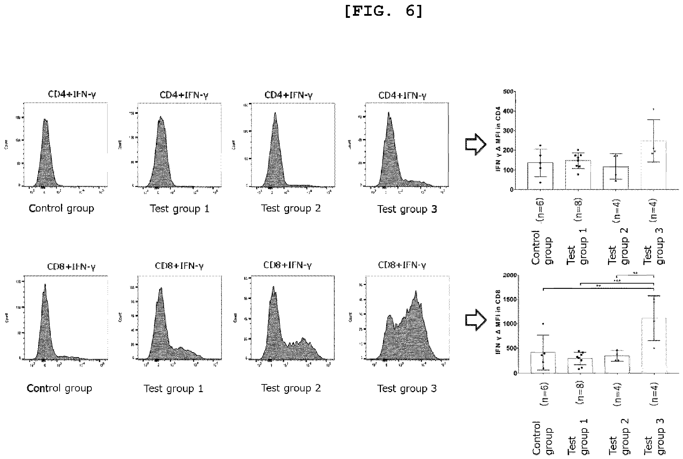

FIG. 6 shows the results of FACS analysis of tumor infiltrating lymphocytes (TIL) after multiple staining by anti-mouse CD4 antibody or instead anti-mouse CD8 antibody and anti-mouse INF-γ antibody.

As seen in the results in FIG. 3, as regards Ki-67 expression of spleen lymphocytes, no conspicuous differences from the untreated group ("control group") could be found in the anti-PD-L1 antibody alone group ("test group 1") or the β-glucan alone group ("test group 2") or the anti-PD-L1 antibody and β-glucan combined group ("test group 3") in both CD4+ T cells and CD8+ T cells.

As seen in the results in FIG. 4, as regards Ki-67 expression of tumor infiltrating lymphocytes, expression tended to be markedly higher in the anti-PD-L1 antibody and β-glucan combined group ("test group 3") in comparison to the untreated group ("control group") or the anti-PD-L1 antibody alone group ("test group 1") or the β-glucan alone group ("test group 2") in both CD4+ T cells and CD8+ T cells (in FIG. 4, "**" indicates p<0.01, "***" indicates <0.001).

As seen in the results in FIG. 5, as regards INF-γ expression of spleen lymphocytes, no conspicuous differences from the untreated group ("control group") could be found in the anti-PD-L1 antibody alone group ("test group 1") or the β-glucan alone group ("test group 2") or the anti-PD-L1 antibody and β-glucan combined group ("test group 3") in both CD4+ T cells and CD8+ T cells.

As seen in the results in FIG. 6, as regards INF-γ expression of tumor infiltrating lymphocytes, expression tended to be markedly higher in the anti-PD-L1 antibody and β-glucan combined group ("test group 3") in comparison to the untreated group ("control group") or the anti-PD-L1 antibody alone group ("test group 1") or the β-glucan alone group ("test group 2") in CD8+ T cells. On the other hand, in CD4+ T cells, virtually no difference could be seen between the untreated group ("control group") and the anti-PD-L1 antibody and β-glucan combined group ("test group 3") (in FIG. 6, "**" indicates p<0.01, "***" indicates p<0.001).

Based on the above, it was clear that administration of anti-PD-L1 antibody and β-glucan activated cell infiltrating T cells and heightened the cytotoxicity thereof. On the other hand, there were no marked effects in spleen lymphocytes. It was also clear that the activating effect was markedly heightened by combined use over administration alone. These results agree well with the tumor volume reduction effect in cancer-bearing mice.

### <Test example 3> (Reference)

Cancer-bearing mice were created by transplantation of a tumor cell line in the same way as in Test example 1, and whether administration of the test substance had an effect on growth of the tumors was studied. In this instance, a β-glucan derived from shiitake ("Micelle Glucan," manufactured by RL-JP Co., Ltd.) (referred to hereinafter as "Micelle β-glucan" hereinafter) was used instead of the β-glucan derived from *Aureobasidium pullulans* as the test substance.

Specifically, cancer-bearing mice were created by inoculating the flanks of C57BL/6 mice (Japan SLC) with 3×10⁵ mouse melanoma cell line B16F10 (Riken Biobank) cells and allowing the tumors to grow for 13 days.

The test animals were divided into four groups: an untreated group, a Micelle β-glucan alone group, an anti-PD-L1 antibody alone group, and an anti-PD-L1 antibody and Micelle β-glucan combined group. The N number was 4 animals in the untreated group, 2 animals in the Micelle β-glucan alone group, 3 animals in the anti-PD-L1 antibody alone group, and 3 animals in the anti-PD-L1 antibody and Micelle β-glucan combined group. Below, for the sake of convenience, the untreated group is sometimes referred to as "control group A," the Micelle β-glucan alone group as "test group 1A," the anti-PD-L1 antibody alone group as "test group 2A," and the anti-PD-L1 antibody and Micelle β-glucan combined group as "test group 3A."

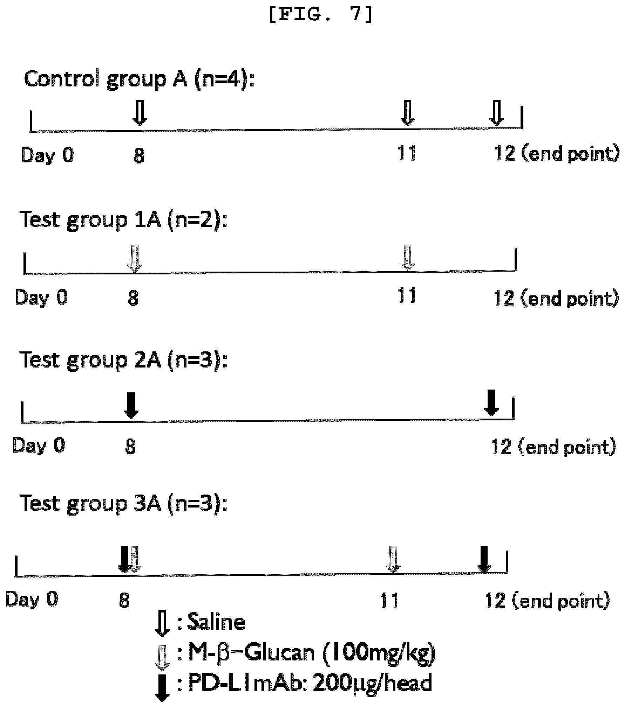

As the form of the test substance administered, in the untreated group ("control group A"), physiological saline was administered intraperitoneally on days 8, 12, and 14 after tumor cell transplantation. In the Micelle β-glucan alone group ("test group 1A"), Micelle β-glucan was administered intraperitoneally in a dosage of 100 mg/kg in terms of the β-glucan amount on days 8 and 11 after tumor cell transplantation. In the anti-PD-L1 antibody alone group ("test group 2A"), anti-mouse PD-L1 antibody (clone: MIH5) was administered intraperitoneally in a dosage of 200 µg/animal on days 8 and 12 after tumor cell transplantation. In the anti-PD-L1 antibody and Micelle β-glucan combined group ("test group 3A"), anti-mouse PD-L1 antibody was administered in the same dosage form as above on days 8 and 12 after tumor cell transplantation and Micelle β-glucan was administered intraperitoneally in a dosage of 100 mg/kg in terms of the β-glucan on days 8 and 11 after tumor cell transplantation. FIG. 7 shows an overview of the administration schedule of each test substance.

The major and minor axes of the tumors were measured on days 6, 8, 11, and 12 after inoculation of the melanoma cell line, and the tumor volume (mm³) was recorded in the same way as in Test example 1.

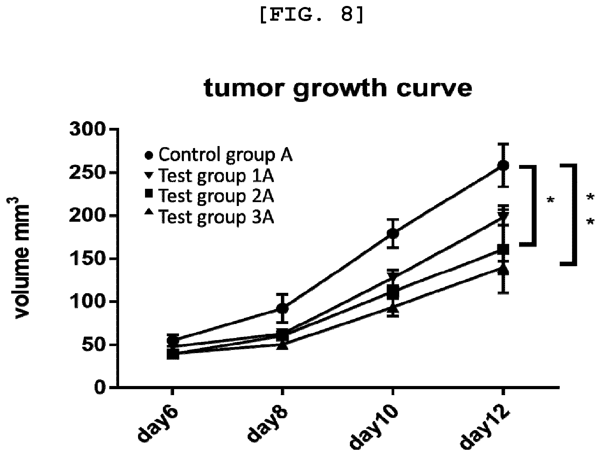

The results on tumor volume after the elapse of the predetermined number of days from tumor cell transplantation are shown in Table 3 an FIG. 8. The results obtained by studying the existence of significance in each group by statistical analysis by the Brown-Forsythe test when comparing tumor volumes on day 12 after tumor cell transplantation are shown in Table 4.

**[Table 3]**

| | Tumor volume (mm³) after elapse of number of days from tumor cell transplantation | | | |
|---|---|---|---|---|
| | Day 6 | Day 8 | Day 10 | Day 12 |
| Control group A | 45.50 | 71.66 | 176.16 | 221.78 |
| | 62.96 | 94.22 | 196.99 | 278.40 |
| | 55.23 | 90.75 | 158.44 | 267.70 |
| | 59.25 | 111.60 | 185.68 | 265.24 |
| Test group 1A | 43.22 | 63.75 | 120.93 | 191.56 |
| | 53.24 | 61.85 | 134.54 | 204.16 |
| Test group 2A | 34.22 | 56.07 | 113.40 | 136.46 |
| | 39.75 | 64.51 | 104.28 | 126.85 |
| | 44.38 | 61.06 | 117.21 | 219.38 |
| Test group 3A | 41.60 | 50.78 | 104.28 | 140.31 |
| | 40.57 | 45.50 | 92.70 | 147.46 |
| | 37.93 | 55.30 | 83.82 | 132.10 |

**[Table 4]**

| Existence of significance in each group when comparing tumor volumes on day 12 after tumor cell transplantation | | | |
|---|---|---|---|
| | Significance | Indication | Adjusted P value |
| Control group A vs test group 2A | Yes | * | 0.0128 |
| Control group A vs test group 3A | Yes | ** | 0.0046 |
| Test group 2A vs test group 3A | No | - | 0.7141 |

In the results, the tumor volume was markedly lower in the anti-PD-L1 antibody and Micelle β-glucan combined group ("test group 3A") in comparison to the untreated group ("control group A"). The reduction effect tended to be higher than in the anti-PD-L1 antibody alone group ("test group 2A") (see comparison on day 12).

## Claims

1. A composition for use in enhancing the effect of an antibody drug in the treatment of cancer, wherein the composition contains a β-glucan derived from *Aureobasidium pullulans* as an active ingredient and is administered together with an antibody drug, and wherein the antibody drug has an effect of suppressing growth of cancer via immune checkpoint inhibition.

2. The composition for use according to Claim 1, wherein the antibody drug includes a monoclonal antibody to PD-L1.

3. The composition for use according to Claim 1 or 2, wherein the cancer is melanoma.

## Patentansprüche

1. Zusammensetzung zur Verwendung bei der Erhöhung der Wirkung eines Antikörpermedikaments bei der Behandlung von Krebs, wobei die Zusammensetzung ein au*s Aureobasidium pullulans* stammendes β-Glucan als Wirkstoff enthält und zusammen mit einem Antikörpermedikament verabreicht wird, und wobei das Antikörpermedikament eine Wirkung zur Unterdrückung des Wachstums von Krebs durch Immun-Checkpoint-Inhibition aufweist.

2. Zusammensetzung zur Verwendung nach Anspruch 1, wobei das Antikörpermedikament einen monoklonalen Antikörper gegen PD-L1 einschließt.

3. Zusammensetzung zur Verwendung nach Anspruch 1 oder 2, wobei der Krebs Melanom ist.

## Revendications

1. Composition destinée à être utilisée pour augmenter l'effet d'un médicament de type anticorps dans le traitement du cancer, dans laquelle la composition contient un β-glucane dérivé d'*Aureobasidium pullulans* comme principe actif et est administrée conjointement avec un médicament de type anticorps, et dans laquelle le médicament de type anticorps présente un effet de suppression de la croissance du cancer par inhibition d'un point de contrôle immunitaire.

2. Composition destinée à être utilisée selon la revendication 1, dans laquelle le médicament de type anticorps inclut un anticorps monoclonal à PD-L1.

3. Composition destinée à être utilisée selon la revendication 1 ou la revendication 2, dans laquelle le cancer est un mélanome.
